Europäisches Patentamt

(19) European Patent Office

Office européen des brevets

(11) Publication number: **0 334 687** .

A1

## EUROPEAN PATENT APPLICATION

(21) Application number: 89400208.8

(22) Date of filing: 25.01.89

(51) Int. Cl.4: **G 01 N 33/74**
G 01 N 33/577, G 01 N 33/68,
G 01 N 33/566, G 01 N 33/564

(30) Priority: 26.01.88 IL 85203

(43) Date of publication of application:
27.09.89 Bulletin 89/39

(84) Designated Contracting States:
AT BE CH DE ES FR GB GR IT LI LU NL SE

(71) Applicant: YEDA RESEARCH & DEVELOPMENT
COMPANY, LIMITED
P.O. Box 95
Rehovot 76100 (IL)

(72) Inventor: **Cohen, Irun Robert**
**11 Hankin Street**
**Rehovot (IL)**

**Elias, Dana**
**57/13 Derech Yavne**
**Rehovot (IL)**

**Shechter, Yoram**
**51 Hanasi Harishon Street**
**Rehovot (IL)**

**Rapoport, Micha**
**4, Ben Gurion Avenue**
**Tel-Aviv (IL)**

(74) Representative: **Rinuy, Santarelli**
**14, avenue de la Grande Armée**
**F-75017 Paris (FR)**

The title of the invention has been amended (Guidelines for Examination in the EPO, A-III, 7.3).

(54) **Immunoassays for incipient diabetes.**

(57) An incipient outbreak of insulin dependent diabetes mellitus can be predicted by testing for the presence of antibodies against idiotypic antibodies which recognize the receptor binding sites of insulin (anti-DM-idiotypic antibodies) or the antibodies against the receptor binding sites of insulin (DM-idiotypic antibodies) in either the serum or urine of the patients to be tested. Monoclonal antibodies are preferably used in such a test. A kit containing such antibodies and conventional adjuvants may also be used.

EP 0 334 687 A1

## Description

## ASSAYS FOR INCIPIENT DIABETES, MONOCLONAL ANTIBODIES USED THEREIN AND KITS FOR USE THEREFOR

### FIELD OF THE INVENTION

The present invention relates to novel assays for detecting in humans an inclination toward, or the initiation of a process leading to diabetes mellitus. The invention further relates to an antibody reagent, polyclonal or preferably monoclonal, and to serum and urine assays based on such antibody reagent enabling a diagnosis of the process of beta cell destruction in humans, thus making it possible to predict an incipient outbreak of insulin dependent diabetes mellitus (IDDM).

### BACKGROUND OF THE INVENTION

Insulin-dependent diabetes mellitus (IDDM) is caused by an autoimmune process which destroys the insulin-producing beta cells which reside in the islets of the pancreas. Diabetes becomes clinically evident only after the vast majority of beta cells are irrevocably destroyed (perhaps 90%) and the life of the individual becomes dependent on an exogenous supply of insulin. In other words, at the time of clinical diagnosis, the autoimmune process has already done irreversible damage, most of it without noticeable symptoms.

Termination of the autoimmune process would result in cure of the disease and prevention of the need for exogenous insulin only if the disease process could be halted while the patient still possessed a sufficient number of beta cells to provide adequate amounts of endogenous insulin. Therefore, any form of therapy of the autoimmune process would be more effective if persons at risk could be identified while they were yet without overt symptoms of disease and thus required insulin replacement for his or her own lost capability to produce insulin.

Mice and other mammals immunized to insulin develop, after a certain period of time (on the order of 7 to 10 days), antibodies to insulin of a specific idiotype which binds to the insulin molecule at the site at which insulin is apparently bound by the insulin (hormone) receptor (Shechter, Y. et al, "Autoantibodies to Insulin Receptor Spontaneously Develop an Anti-Idiotype in Mice Immunized With Insulin", Science. 216, 542-545, 30 April 1982; Shechter, Y. et al, "Mouse Antibodies to the Insulin Receptor Developing Spontaneously on Anti-Idiotypes; I. Characterization of its Antibodies", J. Biol. Chem.., 6416-6415 (1984); Elias, D. et al, "Mouse Antibodies to the Insulin Receptor Developing Spontaneously on Anti-Idiotypes; II. Effects on Glucose Homeostasis and the Insulin Receptor", J. Biol. Chem., 259, 641$_6$ (1984); Cohen, I. R. et al "Immunization to Insulin Generates Anti-Idiotypes that Behave as Antibodies to the Insulin Hormone Receptor and Cause Diabetes Mellitus", in Idiotypes in Biology and Medicine, eds. H. Kohler et al, Academic Press, N.Y., Vol. 20, p. 385 (1984); Shechter, Y. et al in Anti-Idiotypes, Receptors and Molecular Mimicry, eds, Linthicum, D.S. et al, Springer-Verlag, NY, pp. 73-91 (1988)). This operationally defines the DM-idiotypic antibodies. Thus, the insulin-binding site of the DM-idiotypic antibodies mimics the insulin-binding site of the insulin receptor. These same mice were then observed to spontaneously produce antibodies against DM-idiotypic antibodies. Such antibodies against DM-idiotypic anti-insulin antibodies will hereinafter be designated anti-DM-idiotypic antibodies. Thus, in addition to recognizing the DM-idiotype, the anti-DM-idiotypic antibodies also interact with cellular receptors for insulin. Such anti-DM-idiotypic antibodies mimic the structure of the functional part of insulin and behave as antibodies to the insulin receptor.

### SUMMARY OF THE INVENTION

The present invention relates to methods for determining in humans the inclination toward diabetes mellitus. The invention further relates to means for performing assays based on the said methods which comprises use of poly- or preferably monoclonal antibodies and also kits for such assay.

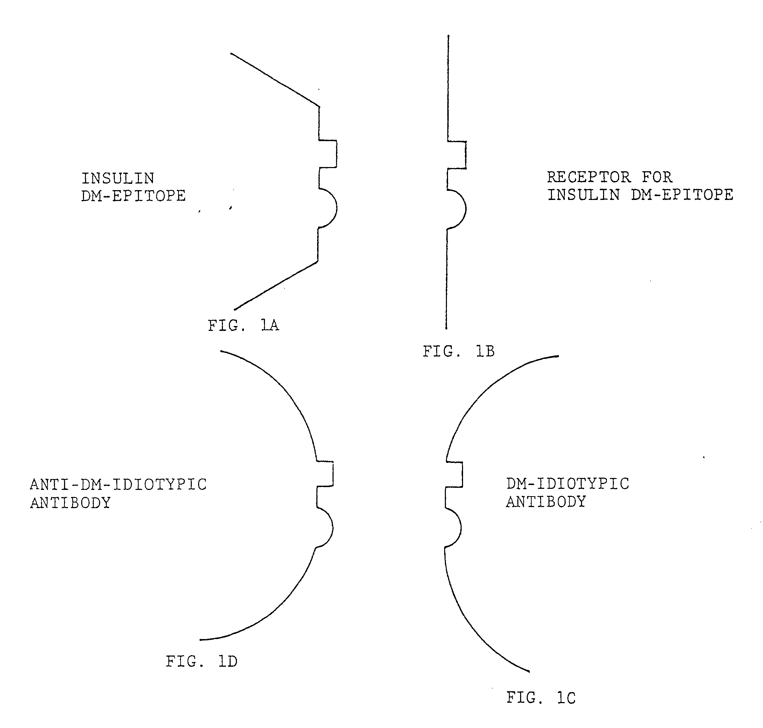

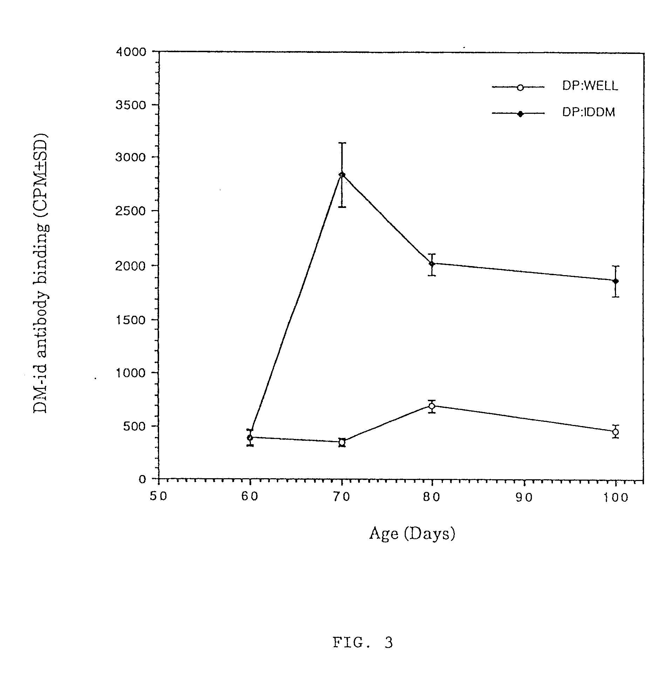

One assay in accordance with the present invention is based on the use of idiotypic antibodies which recognize the receptor binding sites of insulin, herein designated DM-idiotypic antibodies. The assay is used to determine the presence or absence of antibodies against DM-idiotypic antibodies, i.e., anti-DM-idiotypic antibodies, which are recognized by the same DM-idiotypic antibodies as recognize the receptor binding site of insulin. Figure 1 diagrammatically shows the relationships among the hormone, the receptor, the idiotypic antibody and the anti-idiotypic antibody. It can be seen that the DM-idiotypic antibody mimics the insulin DM-epitope receptor site, while the anti-DM-idiotypic antibody mimics the DM-epitope site on the insulin molecule itself. While insulin has many antigenic epitopes, only the DM-epitope by which insulin is bound to the insulin hormone receptor is of interest with respect to the present invention.

Blood of patients with IDDM contains anti-DM-idiotypic antibodies which recognize the insulin binding site of the insulin receptors. Such anti-DM-idiotypic antibodies also recognize polyclonal or monoclonal DM-idiotypic antibodies. In such patients, this anti-DM-idiotypic antibody occupies the insulin receptors and thus prevents directly the attachment of the insulin and/or causes changes in the insulin receptors themselves, thus preventing indirectly the activity of insulin as a hormone.

It has also been discovered that in the early stages of the autoimmune process which leads to the symptoms of clinical IDDM, a relatively large amount of DM-idiotypic antibodies appear in the serum, i.e., substantially larger than the amount of antiDM-idiotypic antibodies. Such an amount of DM-idiotypic anti bodies does not appear in healthy patients. Later in the process, the amount of DM-idiotypic antibodies in the serum greatly decreases and the amount of anti-DM-idiotypic antibodies greatly increases. Thus, the present invention also includes an assay based on the use of anti-DM-idiotypic antibodies to determine the presence or absence of DM-idiotypic antibodies in the serum of the patient being tested. If the titer of either or both of anti-DM-idiotypic antibodies or DM-idiotypic antibodies in the serum of a subject is found to be elevated, this is a positive indication of incipient diabetes.

It has further been discovered that components which bind to either anti-DM-idiotypic antibodies or DM-idiotypic antibodies also appear in the urine of diabetic patients but not in normal urine. With respect to the use of anti-DM-idiotypic antibodies, this means that diabetic urine contains DM-idiotypic antibodies, insulin receptors, and/or other insulin binding molecules, all of which, once established to be present in the urine of newly diagnosed IDDM patients, would be expected also to be present in the urine of patients in the incipient phase of IDDM during destruction of the beta cells. The same is true with respect to the binding of DM-idiotypic antibodies to components found in the urine of diabetic patients but not in normal urine. Usually, the binding of DM-idiotypic antibodies is weaker than that of anti-DM-idiotypic antibodies, although still recognizable. The present invention further relates to means for performing such urine assays, and also kits for such assays.

## BRIEF DESCRIPTION OF THE DRAWINGS

The present invention will be better understood from the following brief description of the drawings and the subsequent detailed description of preferred embodiments.

Figure 1 is a schematic representation of the relation between the hormone (Fig. 1A), its receptor (Fig. 1B), the idiotypic antibody (Fig. 1C) and the anti-idiotypic antibody (Fig. 1D).

Figure 2 is a graph showing the amount of anti-DM-idiotypic antibodies found in the sera of various groups of rats as a function of the age of the rats. The amount of anti-DM-idiotypic antibodies present in the sera was measured by binding to DM-idiotypic antibodies.

Figure 3 is a graph showing the amount of DM-idiotypic antibodies found in the sera of various groups of rats as a function of the age of the rats. The amount of DM-idiotypic antibodies present in the sera was measured by binding to anti-DM-idiotypic antibodies.

Figure 4 is a graph showing the amount of insulin antibodies found in the sera of various groups of rats as a function of the age of the rats. The amount of insulin antibodies present in the sera was measured by binding to insulin.

## DETAILED DESCRIPTION OF PREFERRED EMBODIMENTS

### Production of monoclonal antibodies

A standard method was used to develop monoclonal antiinsulin antibodies. Mice of the (C57BL/6 × BALB/c)F$_1$ hybrid strain were immunized with 25 μg of bovine insulin in complete Freund's adjuvant as described (Shechter, Y. et al (1982), supra; Shechter, Y. et al (1984), supra; Elias, D. et al (1984), supra; Cohen, I. R. et al (1984), supra. Five days later the spleens of two mice were removed and the spleen cells were fused with the HAT sensitive B lymphocyte tumor line SP2/0 using standard procedures to develop hybridomas (Kohler, G. "The Technique of Hybridoma Production", Immunol. Methods, 2, 285-98 (1981)). Of 960 wells tested, 4 were found to be positive for antibodies to insulin. The 4 hybridomas were recloned and their antibodies tested for the DM idiotype. This DM idiotype was detected by its ability to bind specifically to insulin at its receptor binding site and to inhibit the anti-receptor activity of specific anti-idiotypic antibodies.

Binding to native insulin or to acetyl-3-insulin (which has a modified binding site) was assayed using a solid phase radioimmunoassay (RIA). Flexible PVC microtiter plates were coated with 50 μg/ml insulin in PBS, 0.1 ml/well for 1 h at room temperature. Ascites fluid was diluted 1:100 in PBS+0.1% BSA, and added at a concentration of 0.025 ml/well. Plates were washed three times in PBS+0.1% BSA, and $^{125}$I-goat anti-mouse Ig, 100,000 cpm/well was added for 2 h. Plates were then washed four times, dried and the wells were counted on a gamma counter. The ratio of cpm using native insulin to the cpm using modified insulin is presented in Table 1.

Since the anti-DM-idiotypic antibody behaves similarly to insulin, it will cause lipogenesis in adipocytes. The activity of the DM-idiotypic antibody can be measured by its capability of inhibiting the lipogenesis induced by the anti-DM-idiotypic antibody. Lipogenesis was assayed on a suspension of rat adipocytes. Anti-DM-idiotypic serum at 1500 dilution was added to the adipocytes to induce lipogenesis. Anti-insulin monoclonal ascites fluid

3

was added at 1:50, and $^{14}$C-U-glucose was added as a source for lipid synthesis. After 2 h at 37°C the assay was stopped and the radioactivity in the lipid fraction was counted in a beta counter (Shechter, Y. et al (1982), supra; Shechter, Y. et al (1984), supra; Elias, D. et al (1984], supra).

Table 1 illustrates that clones 1D7 and 6D6 were positive for the DM-idiotype. In contrast, clones 14 and 15 were negative for this idiotype in that they failed to distinguish between native insulin and chemically modified insulin which has lost its ability to bind to the insulin receptor. DM positive idiotypic antibodies cannot recognize the modified insulin. Clones 14 and 15 also do not inhibit the activity of the anti-DM-idiotypic antibody (Table 1). Thus, the DM idiotype-positive anti-insulin antibodies (DM-idiotypic antibodies) serve as a reagent to detect anti-idiotypic antibodies to the DM idiotype (anti-DM-idiotypic antibodies).

TABLE 1

Properties of monoclonal anti-insulin antibodies

| Clone | Insulin binding ratio native/modified | % inhibition of anti-idiotypic antibody |
|---|---|---|
| 14 | 1.2 | 0 |
| 15 | 1.2 | 5 |
| 1D7 | 3.8 | 56 |
| 6D6 | 3.1 | 69 |

Hybridoma 1D7 has been deposited at the deposit collection of the Institut Pasteur in Paris, France, under deposit number I-766.

In order to obtain monoclonal anti-DM-idiotypic antibodies, mice of the strain (C57BL/6 × C3H/eB)F$_1$ were immunized to bovine insulin. Twenty-four days later, when the anti-DM-idiotypic antibodies were beginning to appear, the spleen cells of the mice were fused with a standard myeloma partner (SP2/0) in a manner similar to that discussed above for the production of DM-idiotypic antibodies. Five hybridomas producing monoclonal antibodies to DM$^+$-idiotypic antibodies were selected. One of these was designated DT312.

Those of ordinary skill in the art of the production of monoclonal antibodies will readily understand that by repeating the procedure disclosed herein, hybridoma clones can be obtained and selected which also produce antibodies which specifically bind to insulin at its receptor binding site or which specifically bind to DM-idiotypic antibodies and which therefore are intended to be comprehended by the present invention. The specific 1D7 and 6D6 clones disclosed herein are for the purpose of exemplification only. Furthermore, while murine antibodies are used in the specific example herein, it should be understood that any mammal can be selected for immunization and the lymphocytes of such mammal can be hybridized with any compatible myeloma cell line, as long as antibody producing cell lines are obtained. Similarly, any insulin, and particularly human insulin, can be used as the immunizing substance as the insulin receptor binding site does not appear to be species specific. It would also be expected that DM-idiotypic antibody could be used as the immunizing substance as well as insulin to obtain anti-DM-idiotypic clones. Furthermore, the immunoglobulin type of the antibody is immaterial. As long as the DM-idiotypic antibody specifically binds to insulin at its receptor binding site, it is intended to be comprehended by the present invention, and as long as the anti-DM-idiotypic antibody specifically binds to DM idiotypic antibodies, it is intended to be comprehended by the present invention.

There is no evidence to date that the antibodies produced by hybridoma DT312 perform any better or worse than the antibodies produced by any of the four other anti-DM-idiotypic antibody producing hybridomas in the process of the present invention. Thus, it would be expected that any anti-DM-idiotypic antibody would perform as well as those from DT312 and may be substituted therefor in the processes of the present invention using anti-DM-idiotypic antibodies.

Anti-idiotypic antibodies to the DM-idiotype in BB rats

The BB strain of rats is a widely accepted model of human IDDM. Diabetes prone BB rats spontaneously develop an autoimmune attack against their own beta cells resulting in clinical diabetes in about 70% of the rats by about 90 days of age. The remaining 30% of the rats never develop clinical diabetes.

From the diabetes prone strain of BB rats, there was selected a diabetes resistant subline. These rats, in contrast to the diabetes prone line, have a very slight chance of developing IDDM - only a few percent of the rats become diabetic. There were used both the diabetes prone and diabetes resistant lines of BB rats to test whether the presence of anti-DM-idiotypic antibodies in the sera of the rats would be predictive of a future outbreak of clinical IDDM.

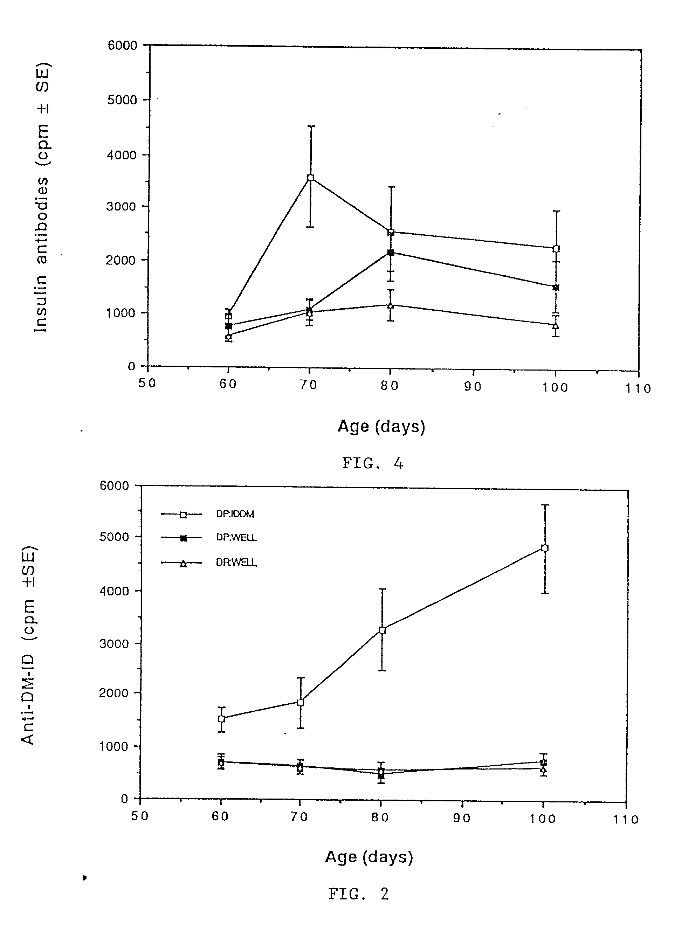

Figure 2 shows the results obtained from sera of rats of various ages related to whether or not the rats eventually developed IDDM. Sera were obtained from groups of 20 to 30 rats each at 60, 70, 80, 90 and 100 days of age and tested in a radioimmunoassay for antibodies to monoclonal DM positive antibody 6D6 as described in Table 1. The open squares ( □ ) designated BB diabetes prone strain rats that went on to develop IDDM by days 90-100. The closed squares (■) designate BB diabetes prone strain rats that did not go on to develop IDDM. The open triangles (Δ) designate BB diabetes resistant strain rats that did not develop IDDM.

It can be seen that the diabetes prone BB rats which eventually developed the disease had increasing amounts of anti-DM-idiotypic antibodies with increasing age. At the time of onset of IDDM, all rats were positive. In contrast, no anti-DM-idiotypic antibodies were found in the diabetes prone and the diabetes resistant rats that did not develop clinical IDDM. One rat of the diabetes resistant strain that actually did develop diabetes was positive in the test. This demonstrates that the presence of anti-DM-idiotypic antibodies is specifically predictive of the development of IDDM in BB rats; all the positive rats went on to develop diabetes, and none of the rats that remained negative developed diabetes. The older the rats, the greater the amount of anti-DM-idiotypic antibodies which was present in the serum.

DM-Idiotypic Antibodies in BB Rats

Additional experiments were conducted to compare the development of DM-idiotypic antibodies and of anti-insulin antibodies (broadly) in the sera of BB/E rats as a function of age and development of IDDM. Sera were obtained from BB/E rats of the diabetes prone (DP) or diabetes resistant (DR) sublines at various ages. Each group contained about 25 sera which were investigated individually by solid phase radioimmunoassay to detect antibodies of the DM-idiotype specificity (using monoclonal anti-DM-idiotypic antibody DT312 as antigen) and antibodies to insulin (using bovine insulin as antigen). Microtiter wells were coated by incubation overnight at 4°C with 0.1 ml of 50 μg/ml of antigen in phosphate buffered saline (PBS). Non-specific binding was blocked by incubating the plates with 1% bovine serum albumin (BSA] in PBS for 1 h at 26 C. Test sera were diluted in 1% DSA at 1:100 and 25 μl were added to duplicate wells and incubated for 2 h at 26°C. The wells were washed x3 in PBS and bound antibody was detected by incubation for 16 h at 4 C with $^{125}$I-rabbit anti-rat Ig. The unbound antibody was washed in PBS x4. The wells were dried and cut for counting using a gamma counter. Figure 3 shows the results using anti-DM-idiotypic antibodies as antigen. Figure 4 shows the results using insulin as antigen. Some of the rats eventually developed IDDM or remained well as indicated. The amount of reactivity is shown as the mean cpm ±SD at various stages. In Figure 3 the closed diamonds (♦) designate BB diabetes prone rats that went on to develop IDDM and the open circles (o) designated diabetes prone BB rats that did not develop IDDM. In Figure 4, the open squares (□) designate BB diabetes prone strain rats that went on to develop IDDM. The closed squares (■) designate BB diabetes prone strain rats that did not go on to develop IDDM. The open triangles (Δ) designate BB diabetes resistant strain rats that did not develop IDDM.

It can be seen that, as indicated in Figure 2, the amount of anti-DM-idiotypic antibody in the serum is definitely indicative of a tendency to develop diabetes. Figure 3 shows that, particularly early in the development of the disease, the amount of DM-idiotypic antibodies in the serum is also predictive of the eventual development of clinical IDDM. However, as can be seen from Figure 4 and from Table 2 hereinbelow, insulin antibodies which are not specific to the DM-idiotype cannot be used as an accurate predictor of incipient diabetes.

Table 2 shows the results obtained in the assay of sera obtained from individual 80 day old BB/E rats of diabetes prone or diabetes resistant sublines. Some of the rats eventually did or did not develop IDDM as indicated. The solid-phase radioimmunoassay was conducted as discussed above for Figures 2 - 4. The sera were considered positive if the mean cpm was 2 or more standard deviations above the mean cpm of normal control sera (about 700 CPM±120).

TABLE 2

DM-id and Anti-DM-id Associated with Development of IDDM in BB/E Rats

| BB/E Rats | Development of IDDM | Incidence of rats with antibodies | | |
| --- | --- | --- | --- | --- |
| | | Total Anti-Insulin | DM-id | Anti-DM-id |
| Diabetes Prone | Yes | 18/24 | 14/24* | 24/24* |
| | No | 6/15 | 2/15 | 0/15 |
| Diabetes Resistant | Yes | 1/1 | 1/1 | 1/1 |
| | No | 7/28 | 0/28 | 0/28 |

* p<0.05 compared to rats that did not develop IDDM

Autoimmune diabetes induced by low-dose streptozotocine: Develop ment of diabetes accompanied by anti-DM-idiotypic antibodies

Streptozotocine is a toxin that causes acute destruction of the beta cells and diabetes when administered to mice at a single dose of 200 mg/kg. Administration of a divided dose of streptozotocine of 40 mg/kg daily for 5 days to male mice of the C57BL/KSJ strain induces a type of autoimmune diabetes (Rossini, A.A. et al, "Immunology of insulin-dependent diabetes mellitus", Ann. Rev. Immunol. 3:289 (1985)). The low dose of toxin apparently partially damages the beta cells such that an autoimmune response is triggered. The autoimmune reaction, and not the streptozotocine itself, is responsible for the diabetic state developing in the C57BL/KSJ mice. Thus, diabetes induced by low dose streptozotocine is a model of autoimmune diabetes that may occur in some humans after a toxic insult of the beta cells.

Similar to the spontaneous diabetes of BB rats, this form of autoimmune diabetes is also associated with the development of anti-DM-idiotypic antibodies. Table 3 shows the results of an experiment in which 20 male C57BL/KSJ mice and 20 male mice of the BALB/c strains, all aged 2.5 months, were treated with the low dose schedule of streptozotocine (40 mg/kg, once daily for 5 days). The development of diabetes was assessed one month after beginning of treatment by detection of hyperglycemia. Blood was collected from each mouse from the tail vein and the concentration of blood glucose was determined. Diabetes was diagnosed by a concentration of greater than 400 mg%. The mouse sera were also examined for the presence of anti-idiotypic antibodies. This was measured by the binding of antibodies in a solid phase radioimmunoassay to guinea pig anti-insulin antibodies which were known to be positive for the DM idiotype (Shechter, Y. et al, (1984), supra; Elias, D. et al, (1984), supra; Cohen, I.R. et al (1984), supra). Table 3 shows that all 20 C57BL/KSJ developed diabetes associated with anti-DM-idiotypic antibodies. In contrast, BALB/c mice which do not develop autoimmune diabetes also did not develop anti-DM-idiotypic antibodies.

TABLE 3

C57BL/KSJ male mice treated with low-dose streptozotocine develop diabetes associated with anti-DM-idiotypic antibodies.

Treatment with streptozotocine (40 mg/kg x 5)

| Mouse strain | Incidence of diabetes | Development of anti-DM-idiotypic antibodies |
|---|---|---|
| C57BL/KSJ | 20/20 | 20/20 |
| BALB/c | 0/20 | 0/20 |

Anti-idiotypic antibodies to the DM-idiotype in humans

To determine whether humans with IDDM are also positive for anti-DM-idiotypic antibodies, there were studied the sera of 30 newly diagnosed untreated IDDM patients and of 20 healthy controls. RIA microtiter plates were coated with 0.25 mg/ml DM-IgG (1D7) (purified from ascites fluid on a Sephacryl S-200 column) overnight at 4°C. Plates were washed, and the human sera, diluted 1:50 in PBS+0.1% BSA, were applied, 25 µl/well and incubated 2 h at room temperature. The plates were washed three times, and $^{125}$I-rabbit anti-human Fab was added, 100,000 cpm/25 µl/well, for 2 h. The plates were washed, dried and the wells cut and counted in a gamma counter. A serum was considered positive if it bound to the monoclonal DM 1D7 idiotype at least twice as much as the mean of healthy controls.

Table 4 shows that 88% of the newly diagnosed IDDM patients were positive for the anti-DM-idiotype while all 20 controls were negative. Sera of IDDM patients and of controls did not react with DM idiotype-negative anti-insulin monoclonal antibodies 14 or 15 (not shown).

Thus, similar to BB rats, humans developing IDDM also develop anti-DM-idiotypic antibodies detectable with the DM-idiotypic monoclonal antibodies.

TABLE 4

Anti-DM-idiotypic antibodies in sera of newly diagnosed IDDM patients

| Sera | Number | % incidence of anti-DM anti-idiotypic antibodies |
|---|---|---|
| Healthy control | 20 | 0 |
| Newly diagnosed IDDM | 38 | 88 |

Sera were obtained from a group of individuals who were siblings of diabetic patients and were thought to be at risk of developing Type 1 diabetes. The patients were followed for 1-6 years and some were found to develop clinical diabetes while others did not. The DM positive monoclonal antibodies 6D6 and 1D7, and polyclonal guinea pig anti-insulin antiserum (Cohen, I. R. et al (1984), supra), were used as the antigens in a solid phase radioimmunoassay to detect the binding of anti-DM-idiotypic human antibodies as described in Shechter, Y. et al (1982), supra; Shechter, Y. et al (1984), supra; Elias, D. et al (1984), supra; and Cohen I. R. et al (1984), supra. Table 5 shows the association between the development of IDDM and the presence of anti-DM-idiotypic antibodies before the outbreak of the disease.

TABLE 5

Association between the presence of anti-DM-idiotypic antibodies and development of IDDM in persons at risk.

| | | | Anti-idiotypic Responses | |
| | | | Monoclonal Anti-insulin DM idiotypes | |
| Subsequent IDDM | Antibody | Guinea pig Polyclonal Anti-insulin | 6D6 | 1D7 |
|---|---|---|---|---|
| Yes | positive | 7/27 | 7/20 | 19/29 |
| | negative | 8/27 | 5/20 | 0/29 |
| No | positive | 3/27 | 2/20 | 4/29 |
| | negative | 9/27 | 6/20 | 6/29 |
| Concordance between Antibody & IDDM | | 16/27(59%) | 13/20(65%) | 25/29(86%) |

It can be seen that the 1D7 monoclonal antibody detected the anti-DM-idiotype with the highest association with subsequent IDDM-concordance of 86%. There were no false negatives (no IDDM patients were anti-DM negative). The 4/29 patients who were positive without IDDM may yet develop the disease. No individuals had detectable anti-idiotypic antibodies to anti-insulin monoclonal antibodies14 or 15 that were not of the DM-idiotype (not shown). It is evident that anti-DM-idiotypic antibodies (detected using the DM positive 1D7 monoclonal antibody) are strongly associated with development of 1DDM.

Urinary Assay of Type I Diabetes Mellitus

A solid phase radioimmunoassay was conducted of urines obtained from 18 newly diagnosed type I diabetic patients and from ten healthy controls. Radioimmunoassay microtiter plates were coated with urinary proteins by incubation overnight at 4°C with test urine at a concentration of protein of 40 µg/ml. Non specific binding was blocked by incubation with 1% bovine serum albumin for 30 minutes at room temperature. The wells are washed and the monoclonal antibodies (purified IgG precipitated from hybridoma culture medium by 45% ammonium sulfate, and purification by filtration on HPLC) were then added at a concentration of 40 µg/ml for 3 hours at room temperature. The wells were washed and the assay developed using $^{125}$I-labeled goat anti mouse Ig, $10^5$ cpm/ml, for two hours at room temperature. The monoclonal antibodies included in this test were the DT312 anti-DM-idiotypic antibody, and, as a control, N13, which is a monoclonal antibody to rat T-lymphocytes. The results are shown in Table 6 as the cpm binding to the wells.

It can be seen from Table 6 that the control monoclonal antibody to rat T-lymphocytes showed background binding to diabetic and to normal urines. However, anti-DM-idiotypic antibodies (DT312) bound to all eighteen diabetic urines and to none of the ten control urines. The mean cpm of binding to the diabetic urines was 36,733 cpm, compared to only 3,091 cpm of binding to the control urine.

TABLE 6

Urinary Assay of Type I Diabetes Mellitus

| Monoclonal Antibody | | Solid Phase Radioimmunoassay | | |
| Designation | Specificity | Urinary Antigen | No. Positive | Mean CPM±SD |
|---|---|---|---|---|
| N13 | anti-T cell | Diabetic | 0/18 | 5,382±2,044 |
| | | Normal | 0/10 | 4,540±1,349 |
| DT312 | anti-DM-idiotype | Diabetic | 18/18 | 36,733±5,850 |
| | | Normal | 0/10 | 3,091± 956 |

It should be noted that the above assay was conducted by coating the solid phase with the urine components followed by incubation with the various monoclonal antibodies. After washing, radiolabeled goat anti-mouse antibodies identify those urine components to which the mouse antibodies have bound. Accordingly, as the anti-DM-idiotypic antibodies (DT312) bind both to DM-idiotypic antibodies and to fragments of the insulin receptor itself, a further test was conducted to determine whether the predominance of the binding was to antibodies or to other urine components.

In this test, either the monoclonal antibodies being tested or insulin, as a control, were first bound to the solid phase, i.e., the microtiter plates were coated with the antibodies, and then the solid phase was incubated with the urine and washed. The binding of urine antibodies to the coating is detected by a second incubation with radiolabelled anti-human Ig. Serum from the same patients were also tested to show a correlation between what circulates in the blood and what leaks into the urine. The results are shown in Table 7.

TABLE 7

Antibodies detected in urine and serum of newly
diagnosed IDDM patients

| Coating on plate | Anti-human Ig detects: | Positive binding (>3 standard deviations above normal mean cpm) | |
|---|---|---|---|
| | | in urine | in serum |
| Insulin | Ab to insulin | 9/21 | 7/21 |
| 1D7 | Anti-DM+-id | 12/21 | 16/21 |
| DT312 | DM+-id | 6/21 | 5/21 |
| 14 | Control for binding other than DM+-id | 0/21 | 0/21 |

In the tests the results of which are reported in Table 7, insulin was coated on the plates by coating the plates for one hour at room temperature with 50 μg/ml of bovine insulin in PBS. Monoclonal antibodies 1D7, DT312 and 14 were coated onto the plates by coating 100 μg/ml of purified IgG (70-80% of total protein is IgG according to SDS-PAGE) overnight at 4°C in PBS. After coating, the plates were washed once with PBS and non-specific binding blocked with 1% BSA in PBS for 1 h, room temperature. Following that, serum was diluted 1:50 in 0.1% BSA in PBS and applied 25 μl/well. Urine was added to the plates undiluted. The plates were incubated 2 h at 37°C, washed x3 and $^{125}$I-anti-human Ig, 100,000 cpm/well, was added for an overnight incubation at 4°C. Nonspecific label was washed x4 and the dried wells were cut and counted.

As the radiolabeled anti-human Ig will not bind to any fragments which have bound to the monoclonal antibodies which are not themselves antibodies, this test only measures the presence of antibodies in the urine and discounts the presence of other fragments to which the various monoclonals may bind.

It can be seen that the results of the urine assays and those of the serum assays are closely, but not perfectly, correlated. Furthermore, it can be seen that not only can the anti-DM-idiotypic antibodies be used for this assay, but also the DM-idiotypic antibodies.

Urine and Serum Assay Correlation

A further test correlating serum and urine results using a solid phase radioimmunoassay in which the plate is coated with the urine was conducted. The results are shown in Table 8.

EP 0 334 687 A1

TABLE 8

Antibodies and shed insulin receptor (or fragments) detected in urine and serum of newly diagnosed IDDM
patients using anti-DM-idiotypic monoclonal antibody in a solid phase radioimmunoassay

| Reagent | Detects | Person | Solid phase assay | |
|---------|---------|--------|-------------------|---|
| | | | Coating on plate | Number positive |
| DT312 | DM+-id and insulin receptors or fragments | IDDM | Urine | 16/18 |
| | | | Serum | 5/18 |
| | | Healthy controls | Urine | 0/20 |
| | | | Serum | 0/20 |
| 1D7 | Anti-DM+-id, insulin | IDDM | Urine | 11/19 |
| | | | Serum | 18/19 |
| | | Healthy controls | Urine | 0/20 |
| | | | Serum | 0/20 |
| 14 | Anti-DM−-id | IDDM | Urine | 0/20 |
| | | | Serum | 0/20 |
| | | Healthy controls | Urine | 0/20 |
| | | | Serum | 0/20 |

In the tests the results of which are reported in Table 8, RIA plates were coated with undiluted urine samples that were dialyzed against 6 changes of PBS, 50 µl urine/well, overnight, at 4°C. Plates were washed once with PBS and nonspecific binding blocked as in Table 7. Mouse anti-DM-idiotypic antibodies (1D7) and DM-idiotypic antibodies (DT312), 25 µl/well, 100 µg/ml, was added for 2 h, 37°C. The rest of the assay was done as described with respect to Table 7 using $^{125}$I-anti-mouse Ig. When serum was coated on plates it was undiluted and undialyzed.

It can be seen that the anti-DM-idiotypic antibodies (DT312) detect more positive in the urine than in the serum. This is probably because the shed insulin receptors are in a very low concentration and cannot be detected in the blood but are concentrated in the urine and can thus be detected. It is further noted that the DM-idiotypic antibodies are useful for testing urine although not as sensitive as the anti-DM-idiotypic antibodies. As the DM-idiotypic antibodies bind more to antibodies than other fragments, the test in which the monoclonal antibodies are bound to the solid phase is more sensitive. On the other hand, the anti-DM-idiotypic antibodies react not only with antibodies in the urine but also with insulin receptors and fragments thereof. Therefore, the assay in which the urine is bound to the solid phase is more sensitive when these antibodies are being used.

Those skilled in the art will be able to devise mechanisms to assay blood and/or urine to obtain the combined results of use of both antibodies (anti-DM-idiotypic and DM-idiotypic). Of course, they cannot both be used in free form as they will react with one another. However, both can be immobilized on separate solid phases, such as on beads, and then combined for incubation with the serum or urine. Such would provide an additive effect, thus improving the number of positives for the diabetic or prediabetic urine or serum while still showing no positives with the healthy controls

Urinary Assay for Incipient Type I Diabetes Mellitus

An identical assay procedure as described for newly diagnosed type I diabetic patients can be conducted on the urine of individuals thought to be at risk of developing Type 1 diabetes, such as siblings of diabetic patients. Those who test positive with DT312 antibodies will go on to develop clinical diabetes, probably within the next one to six years.

In the serum assay experiments discussed above, DM-idiotypic antibodies were used to diagnose anti-DM-idiotypic antibodies in the blood serum of patients (and vice versa), thereby confirming the fact that there was a definite association between the presence of either or both of anti-DM-idiotypic antibodies and DM-idiotypic antibodies, and development of IDDM in persons at risk. This association between the presence of antiDM-idiotypic antibodies and the future development of IDDM was further verified on tests in spontaneously ill BB rats and in C57BL/6/KSJ mice induced by low dose streptozotocine. The rationale for this association is related to the fact that anti-DM-idiotypic antibodies react with receptors for insulin. Therefore, these antibodies might directly attack the beta cells which have insulin receptors, or, more likely, aggravate the destruction of beta cells indirectly by interacting with the insulin receptors on the body's cells and indirectly causing resistance to insulin, thereby leading to overproduction of insulin. Thus, one way or another, they reinforce the process of beta cell failure.

The discovery that some substances in the urine of newly diagnosed IDDM patients bind to anti-DM-idiotypic antibodies and others bind to DM-idiotypic antibodies fits well with the observations with respect to blood serum discussed above and the resulting theories. Only two substances are now known to be recognized by the anti-DM-idiotypic antibodies: DM-idiotypic anti-insulin antibodies and the insulin receptor or fragments thereof. This is inherent in the operational definition of the antibodies used for detection. As anti-DM-idiotypic antibodies and DM-idiotypic antibodies in the serum have been shown to be associated with the development of diabetes, they may leak out into the urine as a consequence of their production and circulation in the blood.

Fragments of the insulin receptor or of other insulin-binding molecules might also be expected to appear in the urine because such molecules are recognized and attacked by the anti-DM-idiotypic antibodies circulating in the blood. The insulin-binding molecules are degraded and fragments leak into the urine. These fragments would also be detectable by the anti-DM-idiotypic antibody reagents of the present invention.

If either or both of the materials discussed above are present in the urine to cause the positive assay results with anti-DM-idiotypic antibodies, this would explain why the urine of patients with incipient IDDM have the same substances as have been shown in the present examples as causing positive results in newly-diagnosed IDDM patients in the serum assay of the present invention. Correlation of the serum and urine results in the same patients has been shown in Tables 7 and 8 above.

To further prove that the urine assay of the present invention is applicable for the detection of incipient diabetes, i.e., prior to the time that symptoms become evident, further tests were conducted using prediabetic NOD mice. The NOD strain of mice is a widely accepted model of human IDDM (Rossini, A. A. et al., "The Immunology of Insulin Dependent Diabetes Mellitus", Ann. Review of Immunology, Vol. 4, 289-320 (1985); Katooka, S. et al., "Immunologic Aspects of the Non-Obese Diabetic (NOD) Mouse", Diabetes, Vol. 32, 247 (1983); Tontesilli, O. et al., "Circulating Lymphocyte Populations and Auto-Antibodies in the Non-Obese Diabetic (NOD) Mice: A Longitudinal Study", Clin.Exp.Immunol., Vol. 70, 84-93 (1987)).

Urine was collected from four NOD mice aged four months, about one to two months prior to the spontaneous onset of overt diabetes. The urine was coated on the solid phase microtiter plates and assays conducted in a manner similar to that discussed above with respect to Table 7. For the purpose of correlation, serum from the same mice was taken as assayed against the DM-idiotypic antibodies and the anti-DM-idiotypic antibodies, using an assay in which the monoclonal antibodies are coated on the plates. The results of both of these tests are shown in Table 9.

TABLE 9

| NOD mouse | Control IgG | Binding of antibodies to immobilized urine substances (cpm) | | | Binding of serum substances to to immobilized antibodies (cpm) | |
|---|---|---|---|---|---|---|
| | | DM⁻-id | DM⁺-id | Anti-DM⁺-id | ($\alpha$-INS) DM⁺-id | ($\alpha$-($\alpha$-INS)) $\alpha$-DM⁺-id |
| 1 | 1,915 | 1,785 | 8,968 | 17,700 | 3,543 | 5,150 |
| 2 | 1,650 | 1,837 | 10,425 | 3,063 | 9,363 | 4,525 |
| 3 | 2,090 | 1,985 | 14,508 | 4,495 | 8,088 | 8,135 |
| 4 | 2,470 | 2,380 | 7,305 | 5,485 | 4,500 | 9,000 |
| Control urine | 2,150 | 1,840 | 1,950 | 1,643 | 1,845 | 2,186 |

11

It can be seen that both tests indicated that each of the four mice tested positive for incident diabetes in both the urine and serum assays using both DM-idiotypic antibodies and anti-DM-idiotypic antibodies, while the control urine tested negative. The DM -idiotypic antibodies also tested negative. These four mice were later observed to develop overt IDDM. This experiment establishes that the urine assays of the present invention, as with the serum assays of the present invention, are operable to detect incipient diabetes prior to the outbreak of overt clinical diabetes.

The Role of Anti-DM-Idiotypic Antibodies and DM-Idiotypic Antibodies in the Development of Type I Diabetes Mellitus

As demonstrated here, the presence of anti-DM-idiotypic antibodies and/or the presence of DM-idiotypic antibodies in either or both of the blood and the urine was associated with the development of autoimmune type 1 diabetes in spontaneously ill BB rats, in C57BL/6/KSJ mice induced by low dose streptozotocine, in prediabetic NOD mice and in human patients. These observations form the basis for the claim that the detection of the anti-DM-idiotype and/or DM-idiotype antibodies in blood or urine may be used to diagnose persons at risk of incipient diabetes. One may ask why these antibodies are associated with diabetes attributed to autoimmune destruction of the beta cells of the pancreatic islets, the producers of insulin. The rationale for this association may be related to the fact that anti-DM-idiotypic antibodies react with receptors for insulin (Shechter, Y. et al (1984), supra; Elias, D. et al (1984), supra; Cohen, I. R. et al (1984), supra). Therefore, these antibodies might directly attack the beta cells which have insulin receptors. However, it is more likely that they aggravate the destruction of beta cells indirectly by interacting with the insulin receptors on the body's cells. As has been demonstrated, the anti-receptor, anti-idiotypic antibodies cause peripheral resistance to insulin by down-regulating and desensitizing the insulin receptors. The peripheral resistance to insulin results in a requirement for increased amounts of insulin. This increased demand for insulin aggravates the damage that the beta cells suffer from the primary autoimmune attack. Thus, a direct autoimmune attack on beta cells, which may be caused by other factors, is combined with peripheral resistance induced by the anti-DM-idiotypic antibodies leading to decompensation of the beta cell function and diabetes. Thus, the anti-DM-idiotypic antibodies are associated with the development of diabetes because they reinforce the process of beta cell failure.

One may speculate on the role of the anti-DM-idiotypic antibodies as follows:

Primary damage to beta cells could be caused by a viral infection or a subclinical toxic insult. The damage triggers an autoimmune process directed against the beta cells. Autoantibodies to insulin develop as the damaged beta cells spill out immunogenic insulin crystals or proinsulin. Some of the antiinsulin antibodies mimic the insulin receptor, that is, they are positive for the DM-idiotype. The $DM^+$-idiotypic antibodies induce the production of anti-DM-idiotypic antibodies. The latter recognize the insulin receptors and by binding to them, cause down-regulation and desensitization. This leads to peripheral insulin resistance and a demand for heightened insulin secretion. The beta cells become overworked as they are forced to produce more insulin and become more susceptible to autoimmune destruction. Thus, the presence of the anti-receptor (anti-DM-idiotypic antibodies) heralds the impending onset of clinical diabetes. As DM-idiotypic antibodies are essentially a precursor for the body's production of anti-DM-idiotypic antibodies, the presence of such DM-idiotypic antibodies would also be expected.

The specific immunoassay technique used in the present examples is radioimmunoassay. It should be understood, however, that once one of ordinary skill in the art becomes aware of the fact that the presence of anti-DM-idiotypic antibodies in the serum of a person, determined by means of assay with DM-idiotypic antibodies, is a positive indication of incipient or existing IDDM, such artisans would be well aware of the types of immunoassay techniques which may be used. Besides radioimmunoassay (RIA solid or liquid phase), any conventional immunoassay technique can be used, such as enzyme-linked immunosorbent assay (ELISA), heterogeneous immunoassay (both competitive and noncompetitive) using labels other than enzymes and radioisotopes, homogeneous immunoassays based on fluorescence quenching and enzyme channeling, immune precipitation (including radial immune diffusion) and agglutination assays based on visual semiquantitative detection or quantitative turbidimetric detection. The assay may use any conventional solid phase or sandwich assay techniques. The specific techniques used to assay for the presence of anti-DM-idiotypic antibodies using DM-idiotypic antibodies or to assay for the presence of DM-idiotypic antibodies using anti-DM-idiotypic antibodies, in blood or urine, are not per se part of the present invention and thus need not be further detailed here.

Similarly, kits may be prepared for carrying out any of the various assays used for accomplishing the present invention. Each such kit would include all of the materials necessary to conduct a single assay or a fixed number of assays. For example, such a kit may contain solid-phase immobilized non-human DM-idiotypic or anti-DM-idiotypic antibodies and a tagged anti-human antibody capable of recognizing the non-variable region of any antibodies which bind to the immobilized antibodies during the course of the assay, such as tagged anti-human Fab. Alternatively, such a kit could contain a solid phase suitable for immobilizing serum or urine, a vial or other container with liquid phase non-human DM-idiotypic or anti-DM-idiotypic antibodies to be used in the assay, and a tagged Ig which binds with all antibodies raised in the animal in which the non-albumin antibodies were raised. The kit should also contain reagent in which immune complexes of DM idiotypic antibodies and anti-DM-idiotypic antibodies can form and may contain directions for using the kit and containers to hold the materials of the kit. Any conventional tag or label may be used, such as a radioisotope, an enzyme, a chromophore or a fluorophore. A typical radioisotope is iodine-125 or sulfur-35. Typical enzymes for this purpose include horseradish peroxidase, α-galactosidase and alkaline phosphatase.

While the invention is described above in relation to certain specific embodiments, it will be understood that many variations are possible, and that alternative materials and reagents can be used without departing from the invention. In some cases such variations and substitutions may require some experimentation, but such will only involve routine testing.

The foregoing description of the specific embodiments will so fully reveal the general nature of the invention that others can, by applying current knowledge, readily modify and/or adapt for various applications such specific embodiments without departing from the generic concept, and therefore such adaptations and modifications are intended to be comprehended within the meaning and range of equivalents of the disclosed embodiments. It is to be understood that the phraseology and terminology employed herein is for the purpose of description and not of limitation.

**Claims**

1. An assay process for determining whether a human has insulin-dependent diabetes mellitus (IDDM) or an inclination toward developing IDDM, comprising assaying the serum or urine of the patient for substances which bind to DM-idiotypic antibodies, capable of binding to the receptor site of insulin, or which bind to anti-DM-idiotypic antibodies, the presence of such substances being indicative of incipient or existing IDDM.

2. An assay in accordance with claim 1, comprising testing for the presence or absence of anti-DM-idiotypic antibodies in the serum of the patient by means of DM-idiotypic antibodies, the presence of anti-DM-idiotypic antibodies being indicative of incipient or existing IDDM.

3. An assay in accordance with claim 1, comprising assaying the serum of the patient for substances which bind to DM-idiotypic antibodies or which bind to anti-DM-idiotypic antibodies, the presence of such substances being indicative of incipient or existing IDDM.

4. An assay in accordance with claim 1, comprising assaying the urine of the patient for substances which bind to DM-idiotypic antibodies or which bind to anti-DM-idiotypio antibodies, the presence of such substances being indicative of incipient or existing IDDM.

5. An assay in accordance with claim 1, comprising assaying the serum of the patient for substances which bind to anti-DM-idiotypic antibodies, the presence of such substances being indicative of incipient or existing IDDM.

6. An assay in accordance with claim 1, comprising assaying the urine of the patient for substances which bind to DM-idiotypic antibodies or which bind to anti-DM-idiotypic antibodies, the presence of such substances being indicative of incipient or existing IDDM.

7. An assay in accordance with claim 1, comprising bringing into contact the serum or urine of the patient and DM-idiotypic antibodies or anti-DM-idiotypic antibodies, and measuring the degree of immunoconjugation of said antibodies with substances in said serum or urine, preferably the said DM-idiotypic or anti-DM-idiotypic antibodies being monoclonal antibodies.

8. A kit for carrying out an assay according to claim 1, comprising DM-idiotypic or anti-DM-idiotypic antibodies, a tagged antibody capable of recognizing the non-variable region of said DM-idiotypic or anti-DM-idiotypic antibodies, reagent capable of permitting immunocomplexation of said antibodies to take place, and containers to hold the materials of the kit.

9. A kit for carrying out an assay according to claim 1, wherein the substances to be detected are antibodies, comprising DM-idiotypic or anti-DM-idiotypic antibodies, a tagged antibody capable of recognizing the non-variable region of the anti-bodies to be detected, reagent capable of permitting immunocomplexation of said antibodies to take place, and containers to hold the materials of the kit.

10. Monoclonal DM-idiotype positive anti-insulin antibodies capable of binding to the receptor site of insulin, preferably of murine origin.

11. Monoclonal anti-DM-idiotypic antibodies capable of binding to the site of DM-idiotype positive anti-insulin antibodies which binds to the receptor site of insulin preferably of murine origin.

12. A method for detecting anti-DM-idiotypic antibodies suspected of being present in a liquid sample, comprising contacting the liquid sample with monoclonal DM-idiotype positive anti-insulin antibodies capable of binding to the receptor site of insulin, and determining the extent of binding, said binding being an indication of the presence of anti-DM-idiotypic antibodies.

13. A method for detecting DM-idiotypic antibodies suspected of being present in a liquid sample, comprising contacting the liquid sample with monoclonal anti-DM-idiotypic antibodies and determining the extent of binding, said binding being an indication of the presence of anti-DM-idiotypic antibodies.

RECEPTOR FOR
INSULIN DM-EPITOPE

FIG. 1B

DM-IDIOTYPIC
ANTIBODY

FIG. 1C

INSULIN
DM-EPITOPE

FIG. 1A

ANTI-DM-IDIOTYPIC
ANTIBODY

FIG. 1D

FIG. 3

FIG. 4

FIG. 2

**European Patent Office**

**EUROPEAN SEARCH REPORT**

Application Number

EP 89 40 0208

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 601 539 (TRUSTEES OF COLUMBIA UNIVERSITY IN THE CITY OF NEW YORK) <br> * Page 16, lines 5-11; page 16, lines 16-19; page 59, lines 2-33 * | 1-7,10-13 | G 01 N 33/74 <br> G 01 N 33/577 <br> G 01 N 33/68 <br> G 01 N 33/566 <br> G 01 N 33/564 |
| X | EP-A-0 133 540 (E.I. DU PONT DE NEMOURS AND CO.) <br> * Page 4, lines 31-34; page 13, lines 20-22; page 15, lines 23-31; page 24, table 1, insulin; page 25, lines 1-30 * | 1-13 | |
| Y | EP-A-0 139 389 (SYNBIOTICS CORP.) <br> * Page 5, lines 13-14; page 7, lines 13-27; page 13, lines 11-30; page 14, example 1; page 22, example 4 * | 1-13 | |
| Y,D | JOURNAL OF BIOLOGICAL CHEMISTRY, vol. 259, no. 10, 26th May 1984, pages 6411-6415, The American Society of Biological Chemists, Inc., Washington, DC, US; Y. SHECHTER et al.: "Mouse antibodies to the insulin receptor developing spontaneously as anti-idiotypes" <br> * Whole article * | 1-13 | |

TECHNICAL FIELDS SEARCHED (Int. Cl.4)

G 01 N

The present search report has been drawn up for all claims

| Place of search | Date of completion of the search | Examiner |
|---|---|---|
| THE HAGUE | 10-05-1989 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)